(19) Europäisches Patentamt
European Patent Office
Office européen des brevets

(11) Veröffentlichungsnummer: **0 313 963 B1**

(12) **EUROPÄISCHE PATENTSCHRIFT**

(45) Veröffentlichungstag der Patentschrift: **23.12.92**

(51) Int. Cl.5: **C07D 409/12**, C07D 405/12, A01N 43/38

(21) Anmeldenummer: **88117315.7**

(22) Anmeldetag: **18.10.88**

Die Akte enthält technische Angaben, die nach dem Eingang der Anmeldung eingereicht wurden und die nicht in dieser Patentschrift enthalten sind.

# (54) N-Phenyltetrahydrophthalimidverbindungen.

(30) Priorität: **27.10.87 DE 3736297**

(43) Veröffentlichungstag der Anmeldung:
**03.05.89 Patentblatt 89/18**

(45) Bekanntmachung des Hinweises auf die Patenterteilung:
**23.12.92 Patentblatt 92/52**

(84) Benannte Vertragsstaaten:
**AT BE CH DE ES FR GB IT LI NL**

(56) Entgegenhaltungen:
**EP-A- 0 233 151**
**WO-A-85/01637**
**GB-A- 2 046 754**

(73) Patentinhaber: **BASF Aktiengesellschaft**
**Carl-Bosch-Strasse 38**
**W-6700 Ludwigshafen(DE)**

(72) Erfinder: **Rueb, Lothar, Dr.**
**Zum Weidentor 4**
**W-6720 Speyer(DE)**
Erfinder: **Eicken, Karl, Dr.**
**Am Huettenwingert 12**
**W-6706 Wachenheim(DE)**
Erfinder: **Zeeh, Bernd, Dr.**
**Oueichstrasse 5**
**W-6703 Limburgerhof(DE)**
Erfinder: **Meyer, Norbert, Dr.**
**Dossenheimer Weg 22**
**W-6802 Ladenburg(DE)**
Erfinder: **Wuerzer, Bruno, Dr.**
**Ruedigerstrasse 13**
**W-6701 Otterstadt(DE)**

**EP 0 313 963 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft N-Phenyltetrahydrophthalimidverbindungen der Formel I,

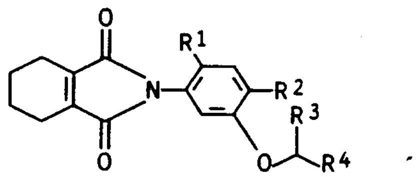

I

in der die Substituenten folgende Bedeutung haben:

$R^1$ Wasserstoff, Fluor oder Chlor

$R^2$ Chlor oder Brom

$R^3$ Wasserstoff oder $C_1$-$C_3$-Alkyl

$R^4$ ein 5-6-gliedriger gesättigter oder ungesättigter Heterocyclus, enthaltend ein Sauerstoff- oder Schwefel-Atom im Ring, der durch bis zu drei $C_1$-$C_3$-Alkylgruppen substituiert sein kann.

Außerdem betrifft die Erfindung ein Verfahren zur Herstellung der Verbindungen I sowie ihre Anwendung als herbizide Mittel.

Aus der Literatur sind N-arylsubstituierte Tetrahydrophthalimide mit Herbizidwirkung bekannt. So werden z.B. in der DE-A 3 013 162 Tetrahydrophthalimide beschrieben, deren Wirksamkeit bei niedrigen Aufwandsmengen unbefriedigend ist.

Der Erfindung lag daher die Aufgabe zugrunde, Verbindungen zu synthetisieren, die bei geringeren Aufwandmengen eine höhere Selektivität gegenüber Kulturpflanzen besitzen.

Entsprechend dieser Aufgabe wurden die N-Tetrahydrophthalimidverbindungen I gefunden, die eine vorteilhafte herbizide Wirkung, besonders im Nachauflaufverfahren, haben und gegenüber einer Reihe von Kulturen selektiv sind.

In speziellen Fällen und bei einigen Kulturpflanzen eignen sich die Verbindungen I auch als Abtrocknungsmittel (Dessicationsmittel) zur Abtötung von grünen Sproßteilen zur Ernteerleichterung.

N-Phenyltetrahydrophthalimidverbindungen der Formel I können beispielsweise dadurch erhalten werden, daß man ein entsprechend substituiertes N-(3-Hydroxyphenyl)-3.4.5.6-tetrahydrophthalimid II bei Temperaturen bis zu 200 °C, vorzugsweise bei 25 bis 150 °C, in einem geeigneten Lösungsmittel in Gegenwart einer Base mit einer geeigneten Verbindung der Formel III umsetzt.

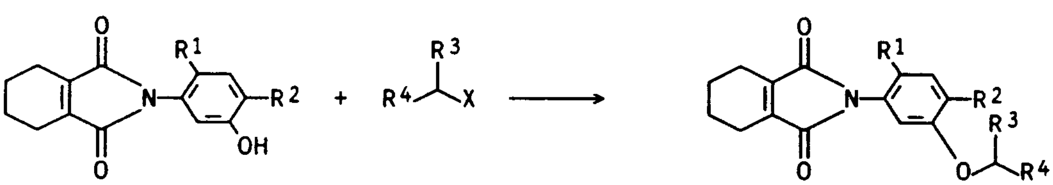

II III I

X in der Formel III bedeutet Halogen wie z.B. Chlor, Brom oder Jod oder Sulfonyloxy wie z.B. Methylsulfonyloxy, Trifluormethylsulfonyloxy, Phenylsulfonyloxy oder Tolylsulfonyloxy, bevorzugt Brom oder Tolylsulfonyloxy.

Man erhält die N-Phenyltetrahydrophthalimidverbindungen der Formel I beispielsweise auch, indem man 3.4.5.6.-Tetrahydrophthalsäureanhydrid mit einem entsprechenden Anilin IV, z.B. in einem Lösungsmittel bei Temperaturen bis 200 °C, vorzugsweise 40 bis 150 °C umsetzt.

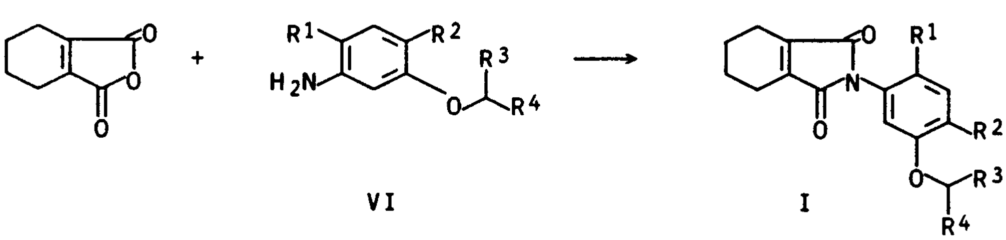

VI I

Als Lösungsmittel eignen sich z.B. niedere Alkansäuren wie Eisessig oder Propionsäure oder aprotische Lösungsmittel wie Toluol oder Xylol in Gegenwart von sauren Katalysatoren wie z.B. aromat. Sulfonsäuren.

Die Anilinderivate VI können z.B. dadurch erhalten werden, daß man eine entsprechend substituierte Nitroverbindung V in Gegenwart von Raney-Nickel oder eines Edelmetallkatalysators wie Platin oder Palladium hydriert oder durch Reduktionsmittel wie Eisen oder ein Zinn(II)-Salz reduziert.

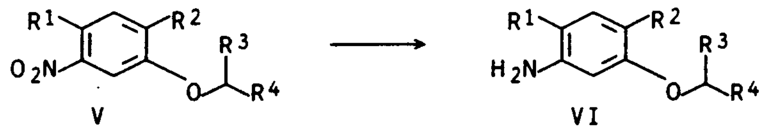

Die Nitroverbindungen V sind durch Umsetzung der entsprechenden Phenole IV mit den Verbindungen der Formel III bei bis zu 200 ° C, vorzugsweise bei 25 bis 150 ° C, in einem aprotisch polaren Lösungsmittel (z.B. Aceton, Acetonitril, Dimethylformamid), in Gegenwart einer Base (z.B. Kaliumcarbonat, Natriumhydroxid, Natriumhydrid) zugänglich.

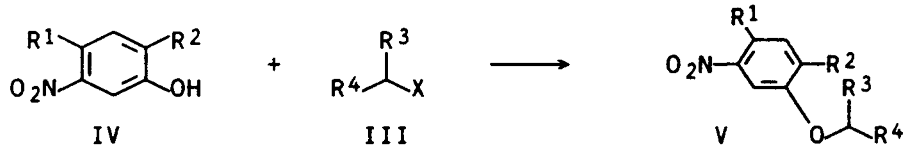

$R^1$ steht bevorzugt für Wasserstoff oder Fluor und $R^2$ bevorzugt für Chlor.

Die Bezeichnung Alkyl umfaßt verzweigte und geradkettige Reste, nämlich Methyl, Ethyl, n-Propyl, Isopropyl.

Die 5-6-gliedrigen Heterocyclen der Formel I sind hydrierte oder teilhydrierte Furan-, Thiophen-, Pyran- und Thiopyran-Abkömmlinge, bevorzugt Tetrahydrofuran, Tetrahydrothiophen, Tetrahydropyran, Dihydropyran, Tetrahydrothiopyran oder Dihydrothiopyran. Falls sie mehrfach substituiert sind, können alle möglichen sterischen Anordnungen vorkommen. Diese können biologisch unterschiedliche Wirkungen haben.

Unter den Verbindungen I sind diejenigen bevorzugt, in denen $R^1$ Wasserstoff oder Fluor, $R^2$ Chlor, $R^3$ Wasserstoff und $R^4$ ein 2- oder 3-Tetrahydrofuranyl, 2- oder 3-Tetrahydrothienyl, 2-, 3- oder 4-Tetrahydropyranyl, 2-, 3- oder 4-Tetrahydrothiopyranyl, 5,6-Dihydro-2H-pyranyl und 5,6-Dihydro-2H-thiopyranyl bedeutet.

Die in den nachstehenden Beispielen wiedergegebenen Arbeitsempfehlungen wurden unter entsprechender Abwandlung der Ausgangsverbindungen zur Gewinnung weiterer Verbindungen der allgemeinen Formel I benutzt. Die Verbindungen sind mit physikalischen Daten in der folgenden Tabelle aufgeführt. Verbindungen ohne solche Angaben lassen sich aus entsprechenden Stoffen in analoger Weise erhalten. Sie lassen aufgrund ihrer nahen strukturellen Beziehung zu den hergestellten und untersuchten Verbindungen eine gleichartige Wirkung erwarten.

Beispiel 1 (Verfahren A)

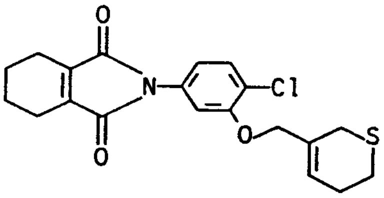

13,9 g N-(4-Chlor-3-hydroxyphenyl)-3.4.5.6-tetrahydrophthalimid, 8,2 g 3-Chlormethyl-5.6-dihydro-2H-thiopyran und 8,3 g Kaliumcarbonat wurden in 150 ml Acetonitril 5 Stunden unter Rückfluß erhitzt. Nach dem Abkühlen wurde abfiltriert, das Filtrat eingeengt und in 200 ml Methylenchlorid aufgenommen, 2 mal mit 10 %iger Natronlauge, 3 mal mit Wasser gewaschen, getrocknet und eingeengt. Man erhielt 15,0 g N-[4-Chlor-3-(5,6-dihydro-2H-thiopyranyl-3-methyloxy)-phenyl]-3.4.5.6-tetrahydrophthalimid (Fp. 116-119 ° C).

(Tab. 1, Nr. 9)

Beispiel 2 (Verfahren B)

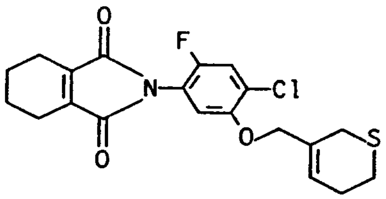

a) 9,6 g 2-Chlor-4-fluor-5-nitrophenol, 8,2 g 3-Chlormethyl-5.6-dihydro-2H-thiopyran und 3,8 g Kaliumcarbonat wurden in 150 ml Acetonitril 5 Stunden unter Rückfluß erhitzt. Nach dem Abkühlen und Abfiltrieren wurde das Filtrat eingeengt und in 200 ml Methylenchlorid aufgenommen. Die organische Phase wurde dreimal mit je 50 ml Wasser gewaschen, getrocknet, eingeengt und mit Petrolether verrührt. Man erhielt 12,5 g 2-Chlor-4-fluor-5-nitro-(5,6-dihydro-2H-thiopyranyl-3-methyloxy)benzol (Fp. 91 - 94 ° C).

b) Zu einer Mischung von 6,7 g Eisenpulver in 50 ml Methanol und 7,5 ml Eisessig gab man bei Rückfluß portionsweise 12,2 g der obigen Nitroverbindung und erhitzte 2 Stunden am Rückfluß. Nach dem Abkühlen wurden 250 ml Wasser zugegeben und abgesaugt. Das Filtrat wurde 3 mal wird je 100 ml Essigester extrahiert, getrocknet und im Vakuum das Lösungsmittel verdampft. Nach Reinigung durch Chromatographie erhielt man 5,5 g 4-Chlor-2-fluor-5-(5,6-dihydro-2H-thiopyranyl-3-methyloxy)-anilin (Fp. 73 - 74 ° C).

c) 5,5 g des obigen Anilins und 3,0 g Cyclohexen-1,2-dicarbonsäureanhydrid wurden in 100 ml Eisessig 5 Stunden unter Rückfluß erhitzt. Nach dem Abkühlen gab man 50 ml Wasser zu und filtrierte ab. Der Niederschlag wurde mit Wasser gewaschen und getrocknet. Man erhielt 6,0 g N-[4-Chlor-2-fluor-5-(5.6-dihydro-2H-thiopyranyl-3-methyloxy)-phenyl]-3.4.5.6-tetrahydrophthalimid (Fp. 134 - 137 ° C) (Tabelle 1 Nr. 10).

Weitere Beispiele für Wirkstoffe, die nach diesen Syntheseprinzipien hergestellt werden können, finden sich in Tabelle 1.

Tabelle 1:

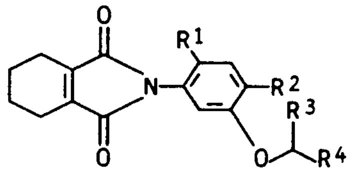 I

| Nr. | $R^1$ | $R^2$ | $R^3$ | $R^4$ | Fp(°C) |
|---|---|---|---|---|---|
| 1 | H | Cl | H | 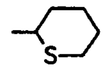 | |
| 2 | F | Cl | H | 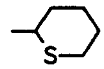 | |
| 3 | H | Cl | H | 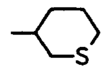 | 143-146 |
| 4 | F | Cl | H | 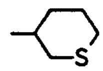 | 162-164 |
| 5 | H | Cl | H | 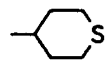 | |
| 6 | F | Cl | H | 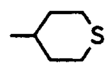 | |
| 7 | H | Cl | H | 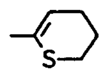 | |
| 8 | F | Cl | H | 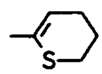 | |
| 9 | H | Cl | H | 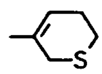 | 116-119 |
| 10 | F | Cl | H | 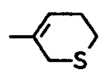 | 134-137 |
| 11 | H | Cl | H | 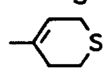 | |
| 12 | F | Cl | H | 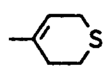 | |
| 13 | H | Cl | H | 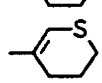 | |
| 14 | F | Cl | H | 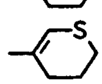 | |
| 15 | H | Cl | H | 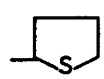 | |
| 16 | F | Cl | H | 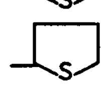 | |
| 17 | H | Cl | H | 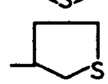 | |
| 18 | F | Cl | H | 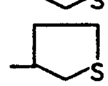 | |

Tabelle 1 - Forts.

| Nr. | $R^1$ | $R^2$ | $R^3$ | $R^4$ | Fp(°C) |
|---|---|---|---|---|---|
| 19 | H | Cl | H | 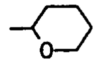 | |
| 20 | F | Cl | H | 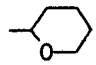 | |
| 21 | H | Cl | H | 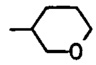 | 108-110 |
| 22 | F | Cl | H | 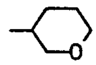 | 104-106 |
| 23 | H | Cl | $CH_3$ | 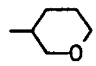 | |
| 24 | F | Cl | $CH_3$ | 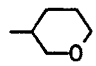 | |
| 25 | H | Cl | H | 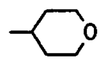 | 140-141 |
| 26 | F | Cl | H | 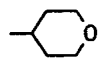 | |
| 27 | H | Cl | H | 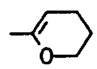 | |
| 28 | F | Cl | H | 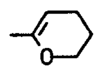 | |
| 29 | H | Cl | H | 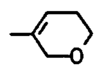 | 90- 92 |
| 30 | F | Cl | H | 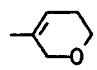 | 131-133 |
| 31 | H | Cl | H | 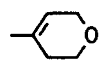 | |
| 32 | F | Cl | H | 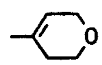 | |
| 33 | H | Cl | H | 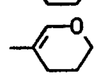 | |
| 34 | F | Cl | H | 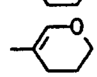 | |
| 35 | H | Cl | H | 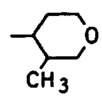 | |
| 36 | F | Cl | H | 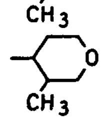 | |
| 37 | H | Cl | H | 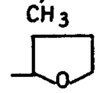 | 74- 76 |
| 38 | F | Cl | H | 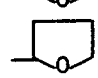 | |
| 39 | H | Cl | H | 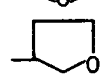 | |
| 40 | F | Cl | H | 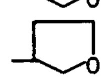 | |

Tabelle 1 - Forts.

| Nr. | $R^1$ | $R^2$ | $R^3$ | $R^4$ | Fp(°C) |
|---|---|---|---|---|---|
| 41 | H | Cl | H | 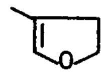 | |
| 42 | F | Cl | H | 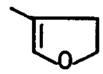 | |
| 43 | H | Cl | $CH_3$ | 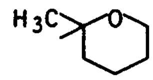 | |
| 44 | F | Cl | $CH_3$ | 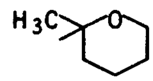 | |
| 45 | H | Cl | $CH_3$ | 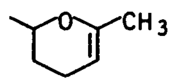 | |
| 46 | F | Cl | $CH_3$ | 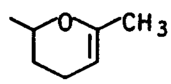 | |

Die Applikation der herbiziden Mittel bzw. der zugrundeliegenden Wirkstoffe (I) kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien 0,005 bis 3,0 vorzugsweise 0,01 bis 0,5 kg/ha.

Die Wirkung der Wirkstoffe der Formel I auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt eingesät.

Zum Zwecke der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmengen für die Nachauflaufbehandlung variieren; sie betragen 0,015 bis 0,125 kg/ha a.S.

Die Versuchsgefäße werden im Gewächshaus aufgestellt, wobei für wärmeliebende Arten wärmere Bereiche (20 bis 35 ° C) und für solche gemäßigter Klimate 10 bis 20 ° C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen gehören den nachstehenden Arten an:

| Abkürzung | Lateinischer Name | Deutscher Name | Englischer Name |
|---|---|---|---|
| ABUTH | Abutilon theophrasti | Chinesischer Hanf | velvet leaf |
| AMARE | Amaranthus spp. | Fuchsschwanzarten | pigweed |
| CHEAL | Chenopodium album | Weißer Gänsefuß | lambsquarters |
| CHYCO | Chrysanthemum coronar. | Wucherblumenarten | marigold |
| GALAP | Galium aparine | Klettenlabkraut | catchweed bedstraw |
| IPOSS | Ipomoea spp. | Prunkwindearten | morningglory |
| LAMAM | Lamium amplexicaule | Stengelumfassende Taubnessel | henbit |
| MERAN | Mercurialis annua | Einjähriges Bingelkraut | annual mercury |
| POLPE | Polygonum persicaria | Flohknöterich | ladystumb |
| SOLNI | Solanum nigrum | Schwarzer Nachtschatten | black nightshade |
| STEME | Stellaria media | Vogelsternmiere | chickweed |
| TRZAS | Triticum aestivum | Sommerweizen | wheat |
| TRZAW | Triticum aestivum | Winterweizen | wheat |
| VERSS | Veronica spp. | Ehrenpreisarten | speedwell |
| VIOAR | Viola arvensis | Stiefmütterchen | violet |

Die Verbindungen 29, 9, 3 und 10 zeigen schon bei geringer Aufwandmenge im Nachauflaufverfahren gute herbizide Wirkung gegen breitblättrige unerwünschte Pflanzen (Tabelle 2).

Unerwünschte breitblättrige Pflanzen werden von den neuen Wirkstoffen 21, 22 und 4 mit 0,03 bzw. 0,06 kg a.S./ha im Nachauflaufverfahren sicher erfaßt.

Weizen erleidet dabei allenfalls geringe und kurzzeitige, sich auswachsende Schäden; diese Herbizide wirken selektiv (Tabellen 3, 4).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (C. canephora, C. liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (G. arboreum, G. herbaceum, G. vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |

| Botanischer Name | Deutscher Name |
|---|---|
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (S. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Wirkstoffe der Formel I sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren, Cyclohexenone, (Hetero)-aryloxy-phenoxy-propionsäure und deren Salze, Ester und Amide und andere in Betracht.

Außerdem kann es von Nutzen sein, die Wirkstoffe der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Tabelle 2 - Herbizide Wirkung gegen unerwünschten Pflanzenwuchs bei Nachauflaufanwendung im Gewächshaus

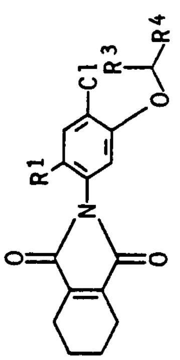

| Bsp. Nr. | R1 | R3 | R4 | kg a.S./ha | Testpflanzen und Schädigung % ABUTH | AMARE | MERAN | SOLNI |
|---|---|---|---|---|---|---|---|---|
| 29 | H | H | 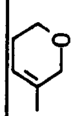 | 0,06 | 100 | 100 | 100 | 100 |
| 9 | H | H | 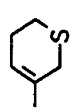 | 0,125 | 100 | 100 | 100 | 100 |
| 3 | H | H | 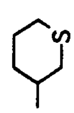 | 0,06 | 100 | 100 | 100 | 100 |
| 10 | F | H | 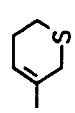 | 0,015 | 100 | 100 | 100 | 100 |

Table 3 - Herbicidal action on unwanted plants and tolerance by a selected crop; postemergence application in the greenhouse

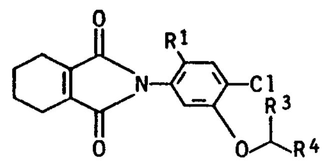

| Ex. no. | $R^1$ | $R^3$ | $R^4$ | kg/ha | Test plants and % damage TRZAS | AMARE | GALAP | IPOSS | STEME | VIOAR |
|---|---|---|---|---|---|---|---|---|---|---|
| 21 | H | H | 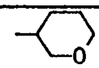 | 0.03 | 10 | 100 | 95 | 95 | 95 | 95 |
| 22 | F | H | 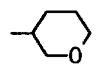 | 0.06 | 10 | 100 | 98 | 100 | 100 | 100 |

Tabelle 4 - Herbizide Wirkung gegen unerwünschte Pflanzen und Verträglichkeit für eine Beispielkultur bei Nachauflaufanwendung im Gewächshaus

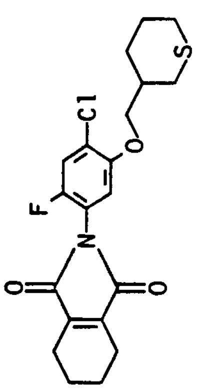

| Bsp. Nr. | kg a.S./ha | Testpflanzen und Schädigung % | | | | | | |
|---|---|---|---|---|---|---|---|---|
| | | TRZAW | CHEAL | CHYCO | IPOSS | LAMAM | POLPE | VERSS |
| 4 | 0,03 | 0 | 100 | 100 | 100 | 98 | 100 | 100 |

## Patentansprüche

## Patentansprüche für folgende Vertragsstaaten : AT, BE, CH, DE, FR, GB, IT, LI, NL

**1.** N-Phenyltetrahydrophthalimidverbindungen der allgemeinen Formel I,

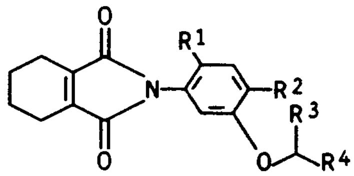

I

in der die Substituenten folgende Bedeutungen haben:

- $R^1$ Wasserstoff, Fluor oder Chlor
- $R^2$ Chlor oder Brom
- $R^3$ Wasserstoff oder $C_1$-$C_3$-Alkyl
- $R^4$ ein 5-6-gliedriger gesättigter oder ungesättigter Heterocyclus, enthaltend ein Sauerstoff- oder Schwefel-Atom, der durch bis zu drei $C_1$-$C_3$-Alkylgruppen substituiert sein kann.

**2.** Verfahren zur Herstellung von Verbindungen der Formel I gemäß Anspruch 1, dadurch gekennzeichnet, daß man ein substituiertes Phenol der Formel II,

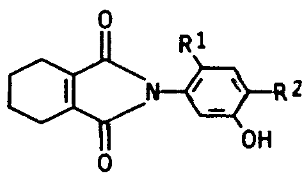

II

mit einer Verbindung der Formel III,

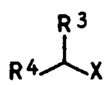

III

worin X Halogen oder Sulfonyloxy bedeutet, in Gegenwart einer Base umsetzt.

**3.** Verfahren zur Herstellung der Verbindungen der Formel I gemäß Anspruch 1, dadurch gekennzeichnet, daß man ein Nitrophenol der Formel IV,

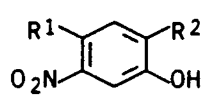

IV

mit einer Verbindung der Formel III, gemäß Anspruch 2,

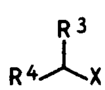

III

zu einem Nitrophenylether der Formel V

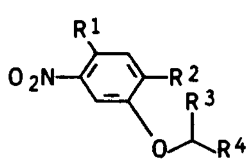

V

umsetzt, anschließend hydriert und das erhaltene Anilin VI

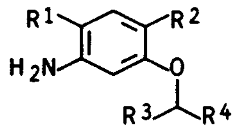

VI

mit Tetrahydrophthalsäureanhydrid zu I umsetzt.

**4.** Verwendung von Verbindungen der Formel I gemäß Anspruch 1 als Herbizide.

**5.** Herbizid wirkendes Mittel, enthaltend eine Verbindung der Formel I gemäß Anspruch 1 und übliche Hilfsstoffe, Streck- und Verdünnungsmittel.

**Patentansprüche für folgenden Vertragsstaat : ES**

**1.** Verfahren zur Herstellung von N-Phenyltetrahydrophthalimidverbindungen der allgemeinen Formel I,

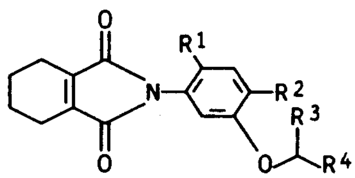

I

in der die Substituenten folgende Bedeutungen haben:

$R^1$ Wasserstoff, Fluor oder Chlor

$R^2$ Chlor oder Brom

$R^3$ Wasserstoff oder $C_1$-$C_3$-Alkyl

$R^4$ ein 5-6-gliedriger gesättigter oder ungesättigter Heterocyclus, enthaltend ein Sauerstoff- oder Schwefel-Atom, der durch bis zu drei $C_1$-$C_3$-Alkylgruppen substituiert sein kann,

dadurch gekennzeichnet, daß man ein substituiertes Phenol der Formel II,

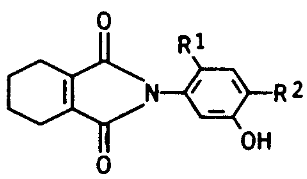

II

mit einer Verbindung der Formel III,

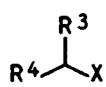

III

worin X Halogen oder Sulfonyloxy bedeutet, in Gegenwart einer Base umsetzt.

**2.** Verfahren zur Herstellung der Verbindungen der Formel I gemäß Anspruch 1, dadurch gekennzeichnet, daß man ein Nitrophenol der Formel IV,

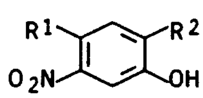

IV

mit einer Verbindung der Formel III, gemäß Anspruch 2,

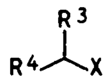

III

zu einem Nitrophenylether der Formel V

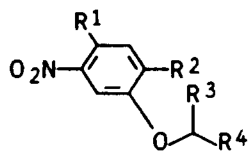

V

umsetzt, anschließend hydriert und das erhaltene Anilin VI

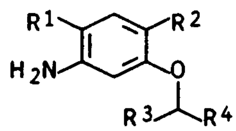

VI

mit Tetrahydrophthalsäureanhydrid zu I umsetzt.

**3.** Verwendung von Verbindungen der Formel I gemäß Anspruch 1 als Herbizide.

**4.** Herbizid wirkendes Mittel, enthaltend eine Verbindung der Formel I gemäß Anspruch 1 und übliche Hilfsstoffe, Streck- und Verdünnungsmittel.

**Claims**

**Claims for the following Contracting States : AT, BE, CH, DE, FR, GB, IT, LI, NL**

**1.** An N-phenyltetrahydrophthalimide compound of the formula I

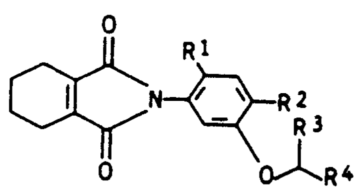

I

where $R^1$ is hydrogen, fluorine or chlorine, $R^2$ is chlorine or bromine, $R^3$ is hydrogen or $C_1$-$C_3$-alkyl and $R^4$ is a 5-membered or 6-membered saturated or unsaturated heterocyclic structure, containing an oxygen or sulfur atom, which may be substituted by up to three $C_1$-$C_3$-alkyl groups.

**2.** A process for the preparation of a compound of the formula I as claimed in claim 1, wherein a substituted phenol of the formula II

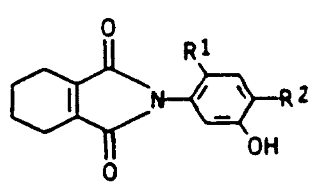

II

is reacted with a compound of the formula III

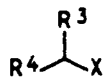

III

where X is halogen or sulfonyloxy, in the presence of a base.

3. A process for the preparation of a compound of the formula I as claimed in claim 1, wherein a nitrophenol of the formula IV

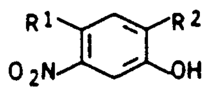

IV

is reacted with a compound of the formula III as claimed in claim 2

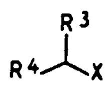

III

to give a nitrophenyl ether of the formula V

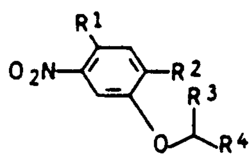

V

the latter is then hydrogenated and the resulting aniline VI

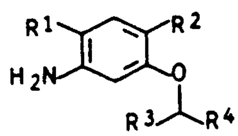

VI

is reacted with tetrahydrophthalic anhydride to give I.

4. Use of a compound of the formula I as claimed in claim 1 as a herbicide.

5. A herbicide containing a compound of the formula I as claimed in claim 1 and conventional additives, extenders and diluents.

**Claims for the following Contracting State : ES**

1. A process for the preparation of an N-phenyltetrahydrophthalimide compound of the formula I

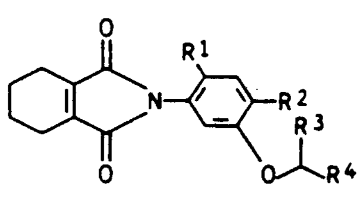

I

where $R^1$ is hydrogen, fluorine or chlorine, $R^2$ is chlorine or bromine, $R^3$ is hydrogen or $C_1$-$C_3$-alkyl and $R^4$ is a 5-membered or 6-membered saturated or unsaturated heterocyclic structure, containing an

oxygen or sulfur atom, which may be substituted by up to three $C_1$-$C_3$-alkyl groups, wherein a substituted phenol of the formula II

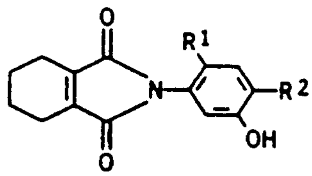

II

is reacted with a compound of the formula III

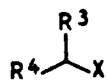

III

where X is halogen or sulfonyloxy, in the presence of a base.

**2.** A process for the preparation of a compound of the formula I as claimed in claim 1, wherein a nitrophenol of the formula IV

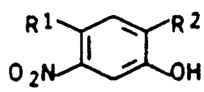

IV

is reacted with a compound of the formula III as claimed in claim 2

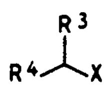

III

to give a nitrophenyl ether of the formula V

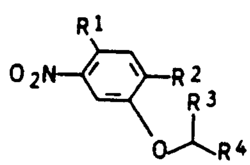

V

the latter is then hydrogenated and the resulting aniline VI

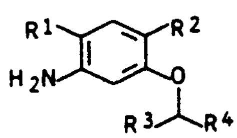

VI

is reacted with tetrahydrophthalic anhydride to give I.

**3.** Use of a compound of the formula I as claimed in claim 1 as a herbicide.

**4.** A herbicide containing a compound of the formula I as claimed in claim 1 and conventional additives, extenders and diluents.

**Revendications**

**Revendications pour les Etats contractants suivants : AT, BE, CH, DE, FR, GB, IT, LI, NL**

**1.** Dérivés de N-phényltétrahydrophtalimide de formule générale I,

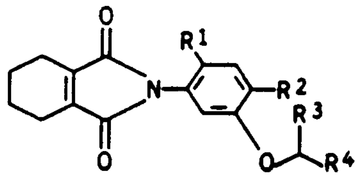

I

dans laquelle les substituants ont les significations suivantes :

$R^1$ hydrogène, fluor ou chlore

$R^2$ chlore ou brome

$R^3$ hydrogène ou alkyle en C1-C3

$R^4$ un hétérocycle saturé ou insaturé à 5 ou 6 chaînons, contenant un atome d'oxygène ou de soufre, qui peut être substitué par jusqu'à trois groupes alkyle en C1-C3.

**2.** Procédé de préparation de composés de formule I, selon la revendication 1, caractérisé par le fait que l'on fait réagir, en présence d'une base, un phénol substitué de formule II

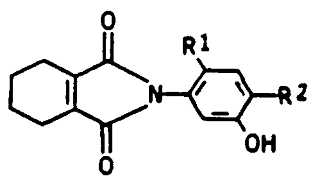

II

avec un composé de formule III,

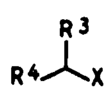

III

dans laquelle X représente halogène ou sulfonyloxy.

**3.** Procédé de préparation des composés de formule I, selon la revendication 1, caractérisé par le fait que l'on fait réagir un nitrophénol de formule IV,

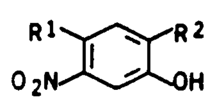

IV

avec un composé de formule III, selon la revendication 2,

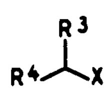

III

pour obtenir un éther nitrophénylique de formule V

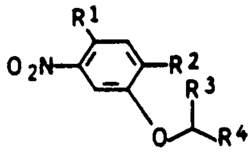

V

on hydrogène ensuite et on fait réagir l'aniline VI résultante

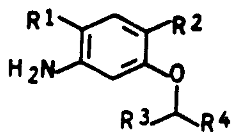

VI

avec de l'anhydride tétrahydrophtalique pour obtenir I.

**4.** Utilisation de composés de formule I, selon la revendication 1, comme herbicides.

**5.** Agent à effet herbicide, contenant un composé de formule I, selon la revendication 1, et des agents auxiliaires, extendeurs et diluants usuels.

**Revendications pour l'Etat contractant suivant : ES**

**1.** Procédé de préparation de dérivés de N-phényltétrahydrophtalimide de formule générale I,

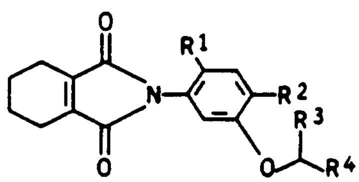

I

dans laquelle les substituants ont les significations suivantes :

- $R^1$ hydrogène, fluor ou chlore
- $R^2$ chlore ou brome
- $R^3$ hydrogène ou alkyle en C1-C3
- $R^4$ un hétérocycle saturé ou insaturé à 5 ou 6 chaînons, contenant un atome d'oxygène ou de soufre, qui peut être substitué par jusqu'à trois groupes alkyle en C1-C3,

caractérisé par le fait que l'on fait réagir, en présence d'une base, un phénol substitué de formule II

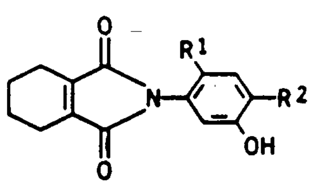

II

avec un composé de formule III,

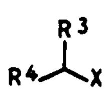

III

dans laquelle X représente halogène ou sulfonyloxy.

**2.** Procédé de préparation des composés de formule I, selon la revendication 1, caractérisé par le fait que l'on fait réagir un nitrophénol de formule IV,

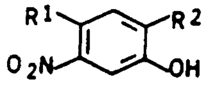

IV

avec un composé de formule III, selon la revendication 1,

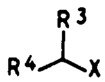

III

pour obtenir un éther nitrophénylique de formule V

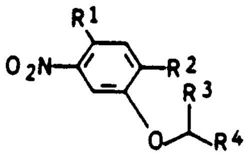

V

on hydrogène ensuite et on fait réagir l'aniline VI résultante

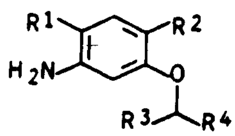

VI

avec de l'anhydride tétrahydrophtalique pour obtenir I.

**3.** Utilisation de composés de formule I, selon la revendication 1, comme herbicides.

**4.** Agent à effet herbicide, contenant un composé de formule I, selon la revendication 1, et des agents auxiliaires, extendeurs et diluants usuels.